(19) Europäisches Patentamt
European Patent Office
Office européen des brevets

(11) **EP 0 960 625 B1**

(12) **EUROPÄISCHE PATENTSCHRIFT**

(45) Veröffentlichungstag und Bekanntmachung des Hinweises auf die Patenterteilung:
**02.01.2003 Patentblatt 2003/01**

(51) Int Cl.$^7$: **A61M 1/34**

(21) Anmeldenummer: **99110193.2**

(22) Anmeldetag: **26.05.1999**

(54) **Sicherheitsvorrichtung für eine Blutbehandlungsvorrichtung**

Safety device for a blood treatment apparatus

Dispositif de sécurité pour un appareil de traitement du sang

(84) Benannte Vertragsstaaten:
**DE ES FR GB IT**

(30) Priorität: **28.05.1998 DE 19823811**

(43) Veröffentlichungstag der Anmeldung:
**01.12.1999 Patentblatt 1999/48**

(73) Patentinhaber: **Fresenius Medical Care Deutschland GmbH**
**61352 Bad Homburg v.d.H. (DE)**

(72) Erfinder:
• **Krämer, Matthias Dr.**
**61381 Friedrichsdorf (DE)**

• **Müller, Carsten Dr.**
**97502 Euerbach (DE)**
• **Johner, Christian Dr.**
**61352 Bad Homburg (DE)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**EP-A- 0 089 003        EP-A- 0 272 414**
**EP-A- 0 358 873        EP-A- 0 686 404**
**DE-A- 4 024 434**

**Beschreibung**

Die Erfindung betrifft eine Sicherheitsvorrichtung für eine extrakorporale Blutbehandlungsvorrichtung, insbesondere eine Hämodialyse-, Hämofiltrationsoder Hämodiafiltrationsvorrichtung.

Zur Entfernung von harnpflichtigen Substanzen und zum Flüssigkeitsentzug werden beim chronischen Nierenversagen verschiedene Verfahren zur operativen Blutreinigung bzw. Blutbehandlung eingesetzt. Bei der Hämodialyse (HD) überwiegt der diffuse Stofftransport, während bei der Hämofiltration (HF) ein konvektiver Stofftransport über die Membran stattfindet. Eine Kombination aus beiden Verfahren ist die Hämodiafiltration (HDF).

Der Entzug von überschüssigem Körperwasser durch Ultrafiltration ist ein wesentlicher Bestandteil der Dialysetherapie. Dieser Vorgang führt meist zu einer Reduktion des Blutvolumens des Patienten. Bei einer zu starken Reduktion des Blutvolumens tritt symptomatische Hypotonie auf, eine häufige Begleiterscheinung der Dialysetherapie. Die Toleranz der Patienten gegen Volumenreduktion ist individuell sehr unterschiedlich; bei kritischen Patientenkollektiven, z.B. Diabetiker, Artherosklerosepatienten, treten die Blutdruckabfälle deutlich gehäuft auf.

Bei der heutigen Standardtherapie wird entweder eine feste Ultrafiltrationsrate UFR oder aber ein fester zeitlicher Verlauf der Rate (UF-Profil) vorgegeben. Eine Messung der Blutvolumenreduktion erfolgt nicht, und erst nach Auftreten von Problemen wird die Ultrafiltrationsrate manuell verringert und evtl. durch Infusionen Blutvolumen substituiert.

Es ist vorgeschlagen worden, das Blutvolumen des Patienten während der extrakorporalen Blutbehandlung zu überwachen und die Ultrafiltrationsrate derart einzustellen, daß Reduktionen des Blutvolumens, die hinsichtlich der Kreislaufstabilität des Patienten nicht tolerierbar sind, vermieden werden. Technische Voraussetzung für dieses Verfahren ist eine hinreichend genaue Sensorik zur Bestimmung des Blutvolumens.

Die Menge des während der Behandlung zu ultrafiltrierenden Volumens, d.h. des Ultrafiltratgesamtvolumens $UFV_{ges}$, sowie die Ultrafiltrationsrate UFR wirken sich, wie bereits erwähnt, auf die Flüssigkeitskompartiments des Körpers sowie auf die Kreislaufregulation aus. Der Vorgang der Ultrafiltration und somit auch die Blutvolumenregelung ist daher sicherheitskritisch. So kann bei unkontrolliertem Flüssigkeitsentzug von einigen l/h bereits nach einigen Minuten ein für die Kreislaufregulation kritisches Blutvolumen erreicht sein und ein schwerer Blutdruckabfall ausgelöst werden, der intensiv medizinische Maßnahmen erforderlich macht.

Bisher konnte nicht sichergestellt werden, daß auch im Falle einer Fehlfunktion der Sensorik und des Regelalogrithmus der Blutvolumenregelung für den Patienten keine Risiken bestehen, die größer als die bei den Standardtherapien vorhandenen Risiken sind, bei denen der Arzt die Verantwortung für die gewählte Ultrafiltrationsrate oder das Ultrafiltrationsprofil übernimmt. Daher ist bisher bei den kommerziell erhältlichen Geräten davon abgesehen worden, die Ultrafiltrationsrate in Abhängigkeit von dem Blutvolumen des Patienten zu regeln.

Die DE-A-40 24 434 beschreibt eine extrakorporale Blutbehandlungsvorrichtung mit einer Ultrafiltrationseinrichtung. Die bekannte Blutbehandlungsvorrichtung verfügt über eine Meßeinheit zum Bestimmen des Blutvolumens, wobei die Blutvolumenbestimmung auf der Grundlage der Messung des Drucks im extrakorporalen Blutkreislauf erfolgt. Die Blutbehandlungsvorrichtung erlaubt die Vorgabe einer bestimmten Blutvolumenabnahme während der Behandlung. Die Ultrafiltration wird so gesteuert, daß die Differenz zwischen der vorgegebenen und der gemessenen Blutvolumenabnahme minimiert wird. Dabei soll ein konventionelles UF-Kontrollsystem die Aufgabe eines Schutzsystems übernehmen und die Überschreitung einer bestimmten UF-Rate verhindern können.

Der Erfindung liegt die Aufgabe zugrunde, eine Sicherheitsvorrichtung fiir eine extrakorporale Blutbehandlungsvorrichtung zu schaffen, die eine Regelung der Ultrafiltrationsrate in Abhängigkeit von dem Blutvolumen ohne Erhöhung des Risikos für den Patienten erlaubt. Die Lösung dieser Aufgabe erfolgt erfindungsgemäß mit den im Patentanspruch 1 angegebenen Merkmalen.

Die Sicherheit des Patienten wird dadurch erhöht, daß die von der Regeleinheit der extrakorporalen Blutbehandlungsvorrichtung in Abhängigkeit von dem Blutvolumen vorgegebene Ultrafiltrationsrate auf einen oberen Grenzwert $UFR_{lim}$ limitiert wird, der aus dem vorgegebenen Ultrafiltratgesamtvolumen $UFV_{ges}$ und der vorgegebenen Behandlungsdauer UFT bestimmt wird. Die Orientierung des oberen Grenzwertes an der Ultrafiltrationsrate und der Behandlungsdauer schafft eine hohe Sicherheit fiir den Patienten, ohne daß der Bereich, in dem die Ultrafiltrationsrate verändert werden kann, zu stark beschränkt wird.

Bei einer bevorzugten Ausführungsform wird durch Bilden des Quotienten aus dem vorgegebenen Ultrafiltratgesamtvolumen $UFV_{ges}$ und der vorgegebenen Behandlungsdauer UFT die mittlere Ultrafiltrationsrate UFRM bestimmt, die zum Entziehen des vorgegebenen Ultrafiltratgesamtvolumens $UFV_{ges}$ innerhalb der Behandlungsdauer UFT erforderlich ist. Der obere Grenzwert $UFR_{lim}$ für die Ultrafiltrationsrate UFR wird dann durch Multiplikation der mittleren Ultrafiltrationsrate UFRM mit einem Faktor $\alpha$ bestimmt. Der Faktor $\alpha$ ist vorzugsweise größer als 1,5 und kleiner als 2,3.

Eine weitere bevorzugte Ausführungsform sieht vor, den Bereich zulässiger Ultrafiltrationsraten zum Ende der Behandlung hin zunehmend zu verkleinern. Eine Verringerung des oberen Grenzwertes für die Ultrafiltrationsrate mit zunehmender Behandlungsdauer ist insbesondere dann vorteilhaft, wenn Regelalgorithmen Verwendung finden, die in Abhängigkeit von dem Blutvolumen am Anfang der Behandlung tendenziell größere Ultrafiltrationsraten als am Behandlungsende vorgeben, so daß die Ultrafiltrationstoleranz erfahrungsgemäß zu Beginn der Behandlung größer als am Behandlungsende ist. Die Verringerung des oberen Grenzwertes für die Ultrafiltrationsrate kann eine explizite Funktion der vergangenen Behandlungszeit oder der bereits ultrafiltrierten Flüssigkeitsmenge sein.

In bevorzugter Ausgestaltung fällt der obere Grenzwert für die Ultrafiltrationsrate während der Behandlungsdauer von einem Wert, der durch Multiplikation der mittleren Ultrafiltrationsrate mit einem Faktor $\alpha$ bestimmt wird, auf die mittlere Ultrafiltrationsrate ab, wobei der Faktor $\alpha$ vorteilhafterweise zwischen 1,5 und 2,3 liegt.

In einer weiteren bevorzugten Ausgestaltung orientiert sich der obere Grenzwert für die Ultrafiltrationsrate $UFR_{lim}$ daran, daß größere Ultrafiltrationsraten dann nicht mehr zugelassen werden sollen, wenn nur noch ein geringes Restvolumen zu entziehen ist. Hierzu wird die Restultrafiltrationsrate $UFRR$ (t) bestimmt, mit der das Ultrafiltratvolumen zu einem bestimmten Zeitpunkt in dem noch zur Verfügung stehenden Zeitraum der Behandlungsdauer $UFT$ entzogen werden kann. Durch Multiplikation der Restultrafiltrationsrate mit einem Faktor $\alpha$, der vorteilhafterweise zwischen 1,5 und 2,3 liegt, wird dann der obere Grenzwert $UFR_{lim}$ (t) zu diesem Zeitpunkt bestimmt. Die Orientierung des oberen Grenzwertes für die Ultrafiltrationsrate an der Restultrafiltrationsrate führt dazu, daß nur dann größere Raten zugelassen werden, wenn diese auch zum Entzug des Restvolumens nötig sind. Die Limitierung wirkt sich im Vergleich zu einem linearen Abfall des oberen Grenzwertes für die Ultrafiltrationsrate insbesondere dann aus, wenn schon ein Großteil des Volumens entzogen ist, also in der kritischen Phase der Annäherung an das Trockengewicht. Es wird hier eine zusätzliche Reduktion der maximal erlaubten Rate bewirkt.

Die Überwachung der von der Regeleinheit der Blutbehandlungsvorrichtung vorgegebenen Ultrafiltrationsrate hat nicht nur zum Ziel, dem Patienten vor unphysiologisch hohen Ultrafiltrationsraten zu schützen, sondern auch dafür Sorge zu tragen, daß der Patient das angestrebte Trockengewicht erreicht. Wenn die Ultrafiltrationsrate auf einen oberen Grenzwert limitiert wird, besteht die Möglichkeit, daß innerhalb der vorgegebenen Behandlungsdauer das zu filtrierende Volumen nicht entzogen werden kann.

Bei einer weiteren bevorzugten Ausführungsform ist daher eine Warneinrichtung vorgesehen, die eine Vergleichseinrichtung zum Vergleichen der Restultrafiltrationsrate $UFRR(t)$ mit dem oberen Grenzwert $UFR_{lim}$ und/oder mit der mittleren Ultrafiltrationsrate $UFRM$ aufweist. Nach dem Überschreiten der mittleren Ultrafiltrationsrate $UFRM$ und/oder des oberen Grenzwertes $UFR_{lim}$ gibt die Warneinrichtung ein Warnsignal ab, mit dem das Pflegepersonal darauf hingewiesen wird, daß Schwierigkeiten im Erreichen des angestrebten Trockengewichts des Patienten bestehen. Der Anwender hat dann die Möglichkeit, die Behandlungsdauer zu verlängern und/oder das Zielvolumen zu senken oder kleinere als ursprünglich geplante Blutvolumina zu tolerieren oder die Behandlung ohne Blutvolumenregelung mit der verbleibenden mittleren Ultrafiltrationsrate zu Ende zu führen.

Da die Limitierung für die Ultrafiltrationsrate nur beim Überschreiten eines unteren Grenzwertes vorgenommen werden sollte, weist die Überwachungseinheit der Sicherheitsvorrichtung vorzugsweise eine Vergleichseinrichtung zum Vergleichen der von der Regeleinheit vorgegebenen Ultrafiltrationsrate mit dem unteren Grenzwert auf. Die Überwachungseinrichtung nimmt nur dann eine Beschränkung der Ultrafiltrationsrate auf den oberen Grenzwert $UFR_{lim}$ vor, wenn die Ultrafiltrationsrate größer als der untere Grenzwert ist.

Im folgenden werden mehrere Ausführungsbeispiele der Erfindung unter Bezugnahme auf die Zeichnungen näher erläutert.

Es zeigen:

Figur 1    eine Hämodiafiltrationsvorrichtung zusammen mit der Sicherheitsvorrichtung in schematischer Darstellung,

Figur 2    den Programmablaufplan, nach dem die Überwachungseinheit der Sicherheitsvorrichtung arbeitet,

Figur 3    den oberen Grenzwert für die Ultrafiltrationsrate $UFR_{lim}$ als Funktion der Behandlungszeit t einer Ausführungsform der Sicherheitsvorrichtung, bei der der obere Grenzwert zum Behandlungsende hin abnimmt,

Figur 4    das Ultrafiltratvolumen $UFV$ als Funktion der Behandlungszeit t im ungünstigsten Fehlerfall unter Ultrafiltrationsratenbegrenzung gemäß der Ausführungsform von Figur 3,

Figur 5    das Ultrafiltratvolumen $UFV$ als Funktion der Behandlungszeit t im ungünstigsten Fehlerfall bei einer weiteren Ausführungsform der Sicherheitsvorrichtung und

Figur 6    die Ultrafiltrationsrate $UFR$, die mittlere Ultrafiltrationsrate $UFRM$ und die Restultrafiltrationsrate $UFRR$ als Funktion der Behandlungszeit t.

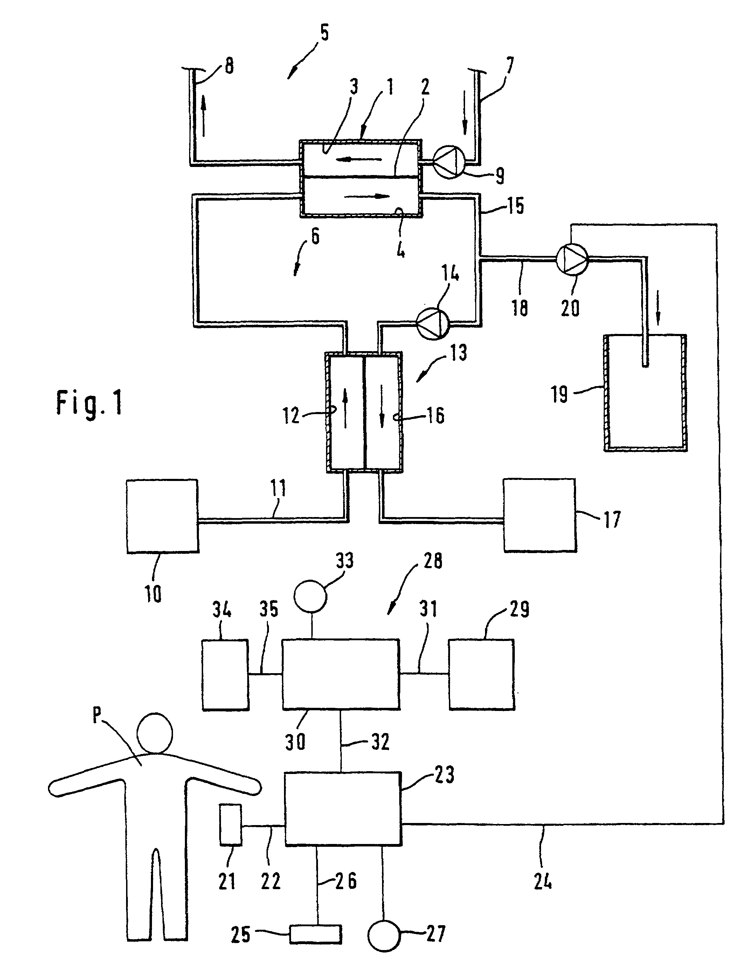

Figur 1 zeigt die wesentlichen Komponenten einer Hämodiafiltrationsvorrichtung zusammen mit der Sicherheitsvorrichtung in schematischer Darstellung. Die Sicherheitsvorrichtung kann Bestandteil der Hämodiafiltrationsvorrichtung oder eine separate Einheit sein, die an eine bestehende Hämodiafiltrationsvorrichtung angeschlossen wird.

Die Hämodiafiltrationsvorrichtung umfaßt einen Dialysator 1, der durch eine semipermeable Membran 2, in eine Blutkammer 3 und eine Dialysierflüssigkeitskammer 4 geteilt ist. Die Blutkammer 3 ist in einen extrakorporalen Blutkreislauf 5 und die Dialysierflüssigkeitskammer 4 in einen Dialysierflüssigkeitskreislauf 6 geschaltet. Von dem Patienten führt eine Blutzuführleitung 7 zu dem Einlaß der Blutkammer 3 des Dialysators 1 und von dem Auslaß der Blutkammer führt eine Blutabführleitung 8 zurück zum Patienten. In die Blutzuführleitung 7 ist eine Blutpumpe 9 geschaltet.

Von einer Dialysierflüssigkeitsquelle 10 führt eine Dialysierflüssigkeitszuführleitung 11 zu dem Einlaß der ersten Bilanzkammer 12 einer Bilanziereinheit 13 und von deren Auslaß zu dem Einlaß der Dialysierflüssigkeitskammer 4 des Dialysators 1.

Eine Dialysierflüssigkeitsabführleitung 15 führt von dem Auslaß der Dialysierflüssigkeitskammer zu der zweiten Bilanzkammer 16 der Bilanziereinheit 13 und von deren Auslaß zu einem Abfluß 17. In die Dialysierflüssigkeitsabführleitung 15 ist stromauf der Bilanziereinheit 13 eine Dialysierflüssigkeitspumpe 14 geschaltet.

Stromauf der zweiten Bilanzkammer 16 der Bilanziereinheit 13 zweigt von der Dialysierflüssigkeitsabführleitung 15 eine Ultrafiltrationsleitung 18 ab, die zu einem Ultrafiltratbehälter 19 führt. Zum Abziehen des Ultrafiltrats ist in die Ultrafiltratleitung 18 eine Ultrafiltratpumpe 20 geschaltet.

Solange die Ultrafiltratpumpe 20 still steht, verhindert die Bilanziereinheit 13 einen Netto-Austausch von Flüssigkeit zwischen Primär- und Sekundärkreislauf 5, 6. Eine Ultrafiltration findet unter diesen Umständen nicht statt. Die Ultrafiltration wird erst durch das Einschalten der Ultrafiltratpumpe in Gang gesetzt, die aus der Dialysierflüssigkeitskammer 4 des Dialysators 1 gezielt Flüssigkeit entzieht (Ultrafiltration).

Die Blutbehandlungsvorrichtung verfügt über eine Meßeinheit 21 zum Bestimmen des Blutvolumens BV des Patienten P während der Hämodiafiltration. Die Meßeinheit 21 ist über eine Datenleitung 22 mit einer Steuereinheit 23 verbunden, die über eine Datenleitung 24 mit der Ultrafiltratpumpe 20 verbunden ist. Im allgemeinen ist es für die erfindungsgemäße Sicherheitsvorrichtung bzw. zur Durchführung des erfindungsgemäßen Verfahrens ausreichend, wenn die Meßeinheit 21 relative Angaben über das Blutvolumen des Patienten P zur Verfügung stellt, wie z.B. Angaben über den Wasseranteil im extrakorporalen Blut des Patienten in der Leitung 7. Es können auch andere mit dem relativen Blutvolumen korrelierende Meßgrößen gemessen werden, wie z.B. die Hämoglobin- oder Proteinkonzentration. Hierzu kann ein Ultraschallsensor Anwendung finden.

Zur Eingabe des Ultrafiltratgesamtvolumens $UFV_{ges}$, das während der gesamten Behandlungsdauer entzogen werden soll, und zur Eingabe der Behandlungsdauer UFT sowie ggf. weiterer patientenspezifischer Parameter ist eine Eingabeeinheit 25 vorgesehen, die über eine Datenleitung 26 mit der Regeleinheit 23 verbunden ist. Die Regeleinheit 23 stellt in Abhängigkeit von dem mit der Meßeinheit 21 gemessenen Blutvolumen BV des Patienten P die Ultrafiltrationsrate UFR(t) der Ultrafiltratpumpe 20 so ein, daß während der vorgegebenen Behandlungsdauer UFT das vorgegebene Ultrafiltratgesamtvolumen $UFV_{ges}$ entzogen wird (Blutvolumenregelung). Hierzu verfügt die Regeleinheit über einen entsprechenden Regelalgorithmus.

Durch Betätigung eines Schalters 27 kann die Regeleinheit 23 deaktiviert und zu einer manuellen Einstellung der Ultrafiltrationsrate übergegangen werden.

Die Sicherheitsvorrichtung 28 verhindert eine Gefährdung des Patienten bei einer fehlerhaften Blutvolumenregelung. Die Sicherheitsvorrichtung umfaßt eine Recheneinheit 29 und eine Überwachungseinheit 30, die über eine Datenleitung 31 miteinander kommunizieren. Die Überwachungseinheit ist über eine Datenleitung 32 mit der Regeleinheit 23 verbunden.

Die Recheneinheit 29 der Sicherheitsvorrichtung 28 bestimmt aus dem vorgegebenen Ultrafiltratgesamtvolumen $UFV_{ges}$ und der vorgegebenen Behandlungsdauer UFT einen oberen Grenzwert für die Ultrafiltrationsrate $UFR_{lim}$.

Durch Betätigung eines Schalters 33 kann die Sicherheitsvorrichtung 28 durch den Anwender deaktiviert werden. Dies ist insbesondere dann sinnvoll, wenn beispielsweise zum Messen von physiologisch relevanten Parametern eine kurzzeitige Erhöhung der Ultrafiltrationsrate erforderlich ist (Ultrafiltrations-Bolus). Dieses Bolusvolumen kann wiederum auf einen oberen Grenzwert beschränkt werden, der von der Überwachungseinheit vorgegeben wird, um die Sicherheit weiter zu erhöhen.

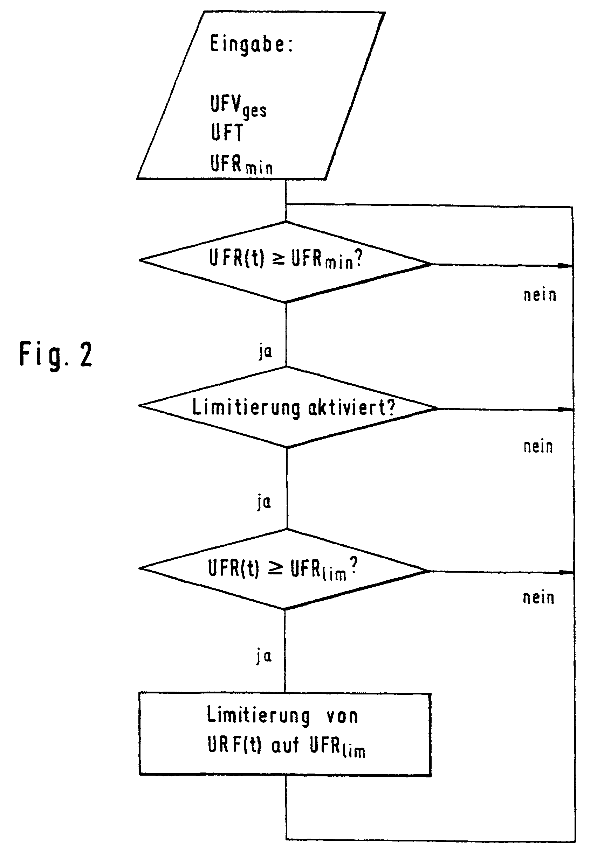

Figur 2 zeigt den Programmablaufplan, nach dem die Überwachungseinheit 29 der Sicherheitsvorrichtung 28 arbeitet.

Zu Beginn der Behandlung werden über die Eingabeeinheit 25 der Hämodiafiltrationsvorrichtung das Ultrafiltratgesamtvolumen $UFV_{ges}$, die Behandlungsdauer UFT und eine minimale Ultrafiltrationsrate $UFR_{min}$ eingegeben. Hierzu kann die Überwachungseinheit auch über eine eigene Eingabeeinheit verfügen. Die patientenspezifischen Parameter $UFV_{ges}$, UFT und $UFR_{min}$ sowie die von der Regeleinheit 23 vorgegebene Ultrafiltrationsrate UFR(t) werden der Überwachungseinheit 29 übermittelt.

Die Überwachungseinheit 29 weist eine Vergleichseinrichtung auf, mit der die von der Regeleinheit 23 vorgegebene Ultrafiltrationsrate UFR(t) mit dem unteren Grenzwert für die Ultrafiltrationsrate $UFR_{min}$ verglichen wird. Ist

UFR(t) kleiner als UFR$_{min}$ findet eine Limitierung der Ultrafiltrationsrate nicht statt. Ist UFR(t) größer als UFR$_{min}$, prüft die Überwachungseinheit, ob die Limitierung der Ultrafiltrationsrate durch den Anwender deaktiviert wurde. Ist die Limitierung aktiviert, bestimmt die Recheneinheit 29 einen oberen Grenzwert für die Ultrafiltrationsrate UFR$_{lim}$, der in der Überwachungseinheit mit der von der Regeleinheit vorgegebenen Ultrafiltrationsrate UFR(t) verglichen wird. Ist UFR(t) größer als UFR$_{lim}$, gibt die Überwachungseinheit als Ultrafiltationsrate UFR(t) den oberen Grenzwert UFR$_{lim}$, vor. Erst wenn die von der Regeleinheit vorgegebene Ultrafiltrationsrate unter den oberen Grenzwert abfällt, beendet die Überwachungseinheit 29 ihren Eingriff in die Regeleinheit 23.

Nachfolgend wird die Bestimmung des oberen Grenzwertes für die Ultrafiltrationsrate UFR$_{lim}$ im einzelnen beschrieben.

Die Recheneinheit 29 der Sicherheitsvorrichtung 28 berechnet aus dem vorgegebenen Ultrafiltratgesamtvolumen UFV$_{ges}$ und der vorgegebenen Behandlungsdauer UFT die mittlere Ultrafiltrationsrate UFRM nach der folgenden Gleichung

$$UFRM = UFV_{ges}/UFT.$$

Bei einer bevorzugten Ausführungsform berechnet die Recheneinheit aus der mittleren Ultrafiltrationsrate UFRM den oberen Grenzwert UFR$_{lim}$ wie folgt:

$$UFR_{lim} = \alpha \_ UFRM,$$

wobei $1,5 < \alpha < 2,3$ ist.

Dieser Grenzwert ist während der Behandlungsdauer konstant. Während der Behandlungsdauer kann der obere Grenzwert UFR$_{lim}$ aber auch auf die mittlere Ultrafiltrationsrate UFRM abfallen.

Bei einer weiteren bevorzugten Ausführungsform berechnet die Recheneinheit den oberen Grenzwert UFR$_{lim}$ nach der folgenden Gleichung:

$$UFR_{lim}(t,\alpha) = [\alpha \cdot UFRM - (\alpha-1) \cdot UFRM \cdot \frac{t}{UFT}] \cdot \Phi(1-\frac{t}{UFT}) + UFRM \cdot \Phi(\frac{t}{UFT}-1)$$

wobei $\Phi(t) = 0$ für $t < 0$ und $\Phi(t) = 1$ für $t \geq 0$ ist.

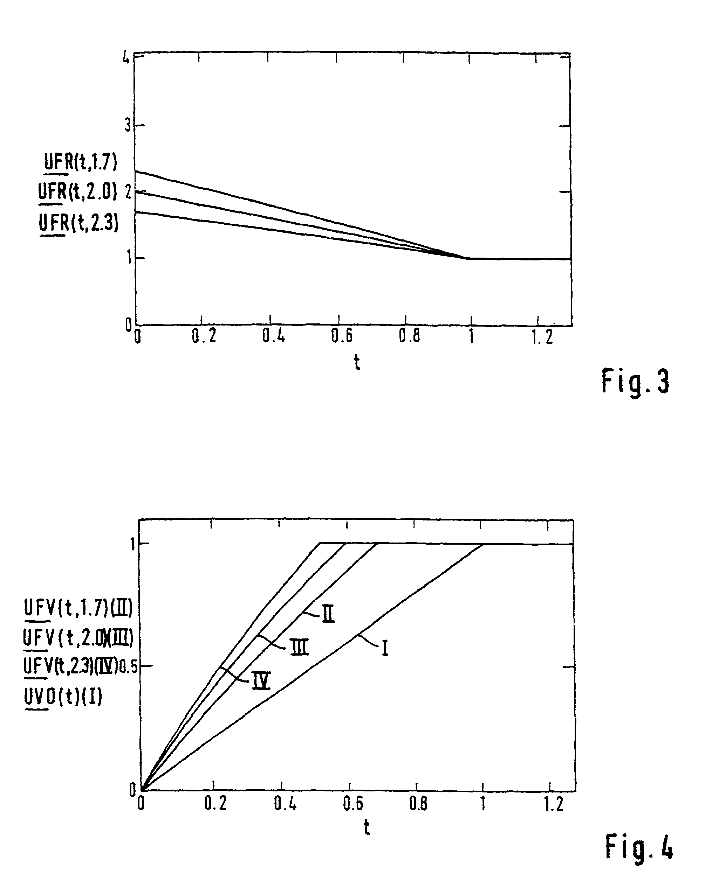

Figur 3 zeigt den oberen Grenzwert der Ultrafiltrationsrate UFR$_{lim}$(t, $\alpha$) als Funktion der Behandlungszeit t, wobei die Ordinatenwerte auf die mittlere Ultrafiltrationsrate UFRM und die Abzissenwerte auf die Behandlungsdauer UFT bezogen sind. Die drei Kurven unterscheiden sich durch den Parameter $\alpha = UFR_{lim}(0, \alpha)/UFRM$. Zu Beginn der Behandlung werden höhere Ultrafiltrationsraten zugelassen (2 bis 2,5 UFRM), gegen Ende der Behandlung wird höchstens die mittlere Ultrafiltrationsrate UFRM zugelassen. Die Wahl eines geeigneten Parameters $\alpha$ wird durch den Variationsbereich für die Ultrafiltrationsrate bestimmt, den der Regelalgorithmus benötigt, mit dem die Regeleinheit 23 arbeitet.

Im ungünstigsten Fehlerfall gibt die Blutvolumenregelung ständig den gerade maximal zulässigen Sollwert nach Figur 3 vor, ohne daß die zugrunde liegende Fehlerursache detektiert wird. Dann stellt sich folgender Verlauf des ultrafiltrierten Volumens als Funktion der Zeit ein:

$$UFV(t,\alpha) = \alpha \bullet UFRM \bullet t - (\alpha-1) \bullet \frac{t^2}{2 \bullet UFT}$$

Die Kurve I in Figur 4 gibt das ultrafiltrierte Volumen bei konstanter Ultrafiltrationsrate (UFR=UFRM) an, wobei die Ordinatenwerte auf das Ultrafiltratgesamtvolumen UFV$_{ges}$ und die Abzissenwerte auf die Behandlungsdauer UFT normiert sind. Im ungünstigsten Fehlerfall steigt das Ultrafiltratvolumen UFV(t, $\alpha$) nach einer der Kurven II, III, IV (abhängig von $\alpha$) in etwa der halben Zeit auf den Endwert 1 an.

Bei einer weiteren bevorzugten Ausführungsform orientiert sich der obere Grenzwert für die Ultrafiltrationsrate UFR$_{lim}$ an der Restultrafiltrationsrate UFRR(t), die in der Recheneinheit nach der folgenden Gleichung berechnet wird:

$$UFRR(t, \alpha) = (UFV_{ges} - UFV(t)) / (UFT-t)$$

Aus der Restultrafiltrationsrate UFRR(t) berechnet die Recheneinheit den oberen Grenzwert wie folgt:

$$UFR_{lim}(t, \alpha) = \alpha\_UFRR(t),$$

wobei $1 < \alpha < 2,3$ ist.

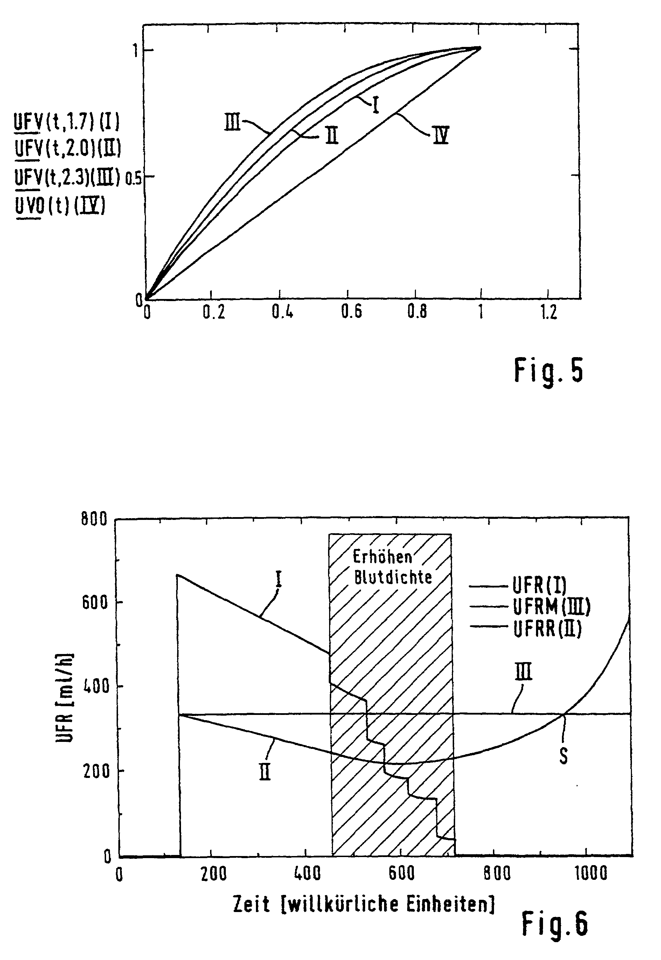

Figur 5 zeigt das Ultrafiltratvolumen $UFV(t, \alpha)$ als Funktion der Behandlungszeit t, wobei die Ordinatenwerte auf das Ultrafiltratgesamtvolumen $UFV_{ges}$ und die Abzissenwerte auf die Behandlungsdauer UFT normiert sind. In Figur 5 ist der im Vergleich zu Figur 4 noch deutlich flachere Anstieg des Ultrafiltratvolumens $UFV(t, \alpha)$ zu erkennen, der sich unter den ungünstigsten Bedingungen beispielsweise bei unerkanntem Ausfall der Blutvolumenregelung zu Beginn der Behandlung ergibt, wenn eine Limitierung der Ultrafiltrationsrate durch die Überwachungseinheit erfolgt.

Neben den obigen Komponenten weist die Sicherheitsvorrichtung noch eine Warneinrichtung 34 auf, die über eine Datenleitung 35 mit der Überwachungseinheit 30 verbunden ist und über zwei akustische und/oder optische Warnsignale verfügt.

Die Warneinrichtung umfaßt eine Vergleichseinrichtung, die die Restultrafiltrationsrate UFRR(t) mit der mittleren Ultrafiltrationsrate UFRM vergleicht. Nach dem Überschreiten der mittleren Ultrafiltrationsrate UFRM gibt die Warneinrichtung ein akustisches und/oder optisches Warnsignal ab, das den Anwender darauf aufmerksam macht, daß innerhalb der Behandlungdauer UFT das vorgegebene Ultrafiltratgesamtvolumen $UFV_{ges}$ möglicherweise nicht entzogen und das Trockengewicht möglicherweise nicht erreicht werden kann.

Die Regeleinheit 23 stellt die Ultrafiltrationsrate UFR(t) so ein, daß UFR(t) größer als die mittlere Ultrafiltrationsrate UFRM ist. Unter normalen Bedingungen verläuft die Restultrafiltrationsrate UFRR(t) dann für die gesamte Behandlungsdauer unterhalb von UFRM. Das Warnsignal wird entweder unmittelbar nach dem Überschreiten der mittleren Ultrafiltrationsrate UFRM, nach Ablauf einer vorbestimmten Zeitdauer von beispielsweise 5 Minuten, nach Überschreiten einer bestimmten Toleranzgrenze UFRM+$\Delta$UFR oder nach Überschreiten einer bestimmten integrierten Ultrafiltrationsmenge abgegeben.

Die Vergleichseinrichtung der Warneinrichtung 34 vergleicht die Restultrafiltrationsrate UFRR(t) auch mit dem oberen Grenzwert der Ultrafiltrationsrate $UFR_{lim}$. Wenn die Restultrafiltrtionsrate UFRR größer als der obere Grenzwert $UFR_{lim}$ ist, gibt die Warneinrichtung ein anderes akustisches und/oder optisches Warnsignal ab, das den Anwender darauf hinweist, daß das Trockengewicht bei aktivierter Überwachungseinheit nicht mehr erreicht werden kann.

Figur 6 zeigt den Fall, daß die Regeleinheit beispielsweise aufgrund eines Fehlers während der Behandlung (schraffierter Bereich) eine sehr geringe Ultrafiltrationsrate UFR vorgibt (Kurve I). In diesem Fall steigt die Restultrafiltrationsrate UFRR an, die zum Entzug des noch verbleibenden Volumens in der restlichen Behandlungszeit notwendig ist (Kurve II). Der Schnittpunkt zwischen der Restultrafiltrationsrate UFRR und der mittleren Ultrafiltrationsrate UFRM (Kurve III) ist in Figur 6 mit S bezeichnet.

**Patentansprüche**

1. Sicherheitsvorrichtung fiir eine extrakorporale Blutbehandlungsvorrichtung, die aufweist

eine Blutzuführleitung (7), die von dem Patienten zu dem Einlaß einer ersten Kammer (3) einer durch eine semipermeable Membran (2) in die erste und eine zweite Kammer (3, 4) getrennte Austauscheinheit (1) fühlt, und einer Blutabführleitung (8), die von einem Auslaß der ersten Kammer zum Patienten führt,

eine Ultrafiltrationseinrichtung (18, 19, 20) zum Entziehen eines vorgegebenen Ultrafiltratgesamtvolumens $UFV_{ges}$ aus der zweiten Kammer der Austauscheinheit mit einer Ultrafiltrationsrate UFR(t) während einer vorgegebenen Behandlungsdauer UFT,

eine Meßeinheit (21) zum Bestimmen des Blutvolumens BV oder einer mit dem Blutvolumen korrelierenden Meßgröße des Patienten und

eine Regeleinheit (23) zur Vorgabe einer Ultrafiltrationsrate UFR(t) in Abhängigkeit von dem Blutvolumen BV, wobei die Sicherheitsvorrichtung (28) aufweist:

eine Einheit (29) zur Vorgabe eines oberen Grenzwertes fiir die Ultrafiltrationsrate UFR(t) und eine Überwachungseinheit (30) zum Begrenzen der von der Regeleinheit (23) vorgegebenen Ultrafiltrationsrate UFR(t) auf den oberen Grenzwert $UFR_{lim}$, wenn die von der Regeleinheit vorgegebene Ultrafiltrationsrate UFR(t) den oberen Grenzwert $UFR_{lim}$ erreicht, **dadurch gekennzeichnet, daß** die Einheit zur Vorgabe eines oberen Grenzwertes für die Ultrafiltrationsrate UFR(t) eine Recheneinheit ist, die derart ausgebildet ist, daß der obere Grenzwert aus dem vorgegebenen Ultrafiltratgesamtvolumen $UFV_{ges}$ und der vorgegebenen Behandlungsdauer UFT bestimmbar ist.

**2.** Sicherheitsvorrichtung nach Anspruch 1, **dadurch gekennzeichnet, daß** die Recheneinheit (29) derart ausgebildet ist, daß durch Bilden des Quotienten aus dem vorgegebenen Ultrafiltratgesamtvolumen $UFV_{ges}$ und der vorgegebenen Behandlungsdauer UFT die mittlere Ultrafiltrationsrate UFRM, die zum Entziehen des vorgegebenen Ultrafiltratgesamtvolumens $UFV_{ges}$ innerhalb der Behandlungsdauer UFT erforderlich ist, und durch Multiplikation der mittleren Ultrafiltrationsrate UFRM mit einem Faktor $\alpha$ der obere Grenzwert $UFR_{lim}$ bestimmbar ist.

**3.** Sicherheitsvorrichtung nach Anspruch 1, **dadurch gekennzeichnet, daß** die Recheneinheit (29) derart ausgebildet ist, daß ein mit zunehmender Behandlungsdauer abnehmender oberer Grenzwert für die Ultrafiltrationsrate $UFR_{lim}(t)$ bestimmbar ist.

**4.** Sicherheitsvorrichtung nach Anspruch 1, **dadurch gekennzeichnet, daß** die Recheneinheit (29) derart ausgebildet ist, daß ein mit zunehmendem Ultrafiltratvolumen UFV abnehmender oberer Grenzwert fiir die Ultrafiltrationsrate $UFR_{lim}(t)$ bestimmbar ist.

**5.** Sicherheitsvorrichtung nach Anspruch 3 oder 4, **dadurch gekennzeichnet, daß** die Recheneinheit (29) derart ausgebildet ist, daß durch Bilden des Quotienten aus dem vorgegebenen Ultrafiltratgesamtvolumen $UFV_{ges}$ und der vorgegebenen Behandlungsdauer UFT die mittlere Ultrafiltrationsrate UFRM, die zum Entziehen des vorgegebenen Ultrafiltratgesamtvolumens $UFV_{ges}$ innerhalb der Behandlungsdauer UFT erforderlich ist, und nach der folgenden Gleichung der obere Grenzwert $UFR_{lim}(t, \alpha)$ zu einem bestimmten Zeitpunkt t bestimmbar ist,

$$UFR_{lim}(t,\alpha)=[\alpha \cdot UFRM-(\alpha-1) \cdot UFRM \cdot \frac{t}{UFT}] \cdot \Phi(1-\frac{t}{UFT})+UFRM \cdot \Phi(\frac{t}{UFT}-1)$$

wobei $\Phi(t)=0$ für $t<0$ und $\Phi(t)=1$ für $t \geq 0$ ist.

**6.** Sicherheitsvorrichtung nach Anspruch 1, **dadurch gekennzeichnet, daß** die Recheneinheit (29) derart ausgebildet ist, daß aus dem vorgegebenen Ultrafiltratgesamtvolumen $UFV_{ges}$ und der vorgegebenen Behandlungsdauer UFT nach der folgenden Gleichung

$$UFRR(t) = (UFV_{ges}-UFV(t)) / (UFT-t)$$

die Restultrafiltrationsrate UFRR(t) bestimmbar ist, mit der das Ultrafiltratvolumen UFV zu einem bestimmten Zeitpunkt in dem noch zur Verfügung stehenden Zeitraum der Behandlungsdauer UFT entzogen werden kann, und daß durch Multiplikation der Restultrafiltrationsrate UFRR(t) mit einem Faktor $\alpha$ der obere Grenzwert $UFR_{lim}(t, \alpha)$ zu diesem Zeitpunkt t bestimmbar ist.

**7.** Sicherheitsvorrichtung nach Anspruch 6, **dadurch gekennzeichnet, daß** eine Warneinrichtung (34) vorgesehen ist, die eine Vergleichseinrichtung zum Vergleichen der Restultrafiltrationsrate UFRR(t) mit der mittleren Ultrafiltrationsrate UFRM aufweist, wobei die Warneinrichtung derart ausgebildet ist, daß nach Überschreiten der mittleren Ultrafiltrationsrate UFRM ein Warnsignal abgegeben wird.

**8.** Sicherheitsvorrichtung nach Anspruch 6, **dadurch gekennzeichnet, daß** eine Warneinrichtung (34) vorgesehen ist, die eine Vergleichseinrichtung zum Vergleichen der Restultrafiltrationsrate UFRR(t) mit dem oberen Grenzwert $UPR_{lim}$ für die Ultrafiltrationsrate aufweist, wobei die Warneinrichtung derart ausgebildet ist, daß nach Überschreiten des oberen Grenzwertes $UFR_{lim}$ ein Warnsignal abgegeben wird.

**9.** Sicherheitsvorrichtung nach einem der Ansprüche 1 bis 8, **dadurch gekennzeichnet, daß** die Überwachungseinheit (30) eine Vergleichseinrichtung zum Vergleichen der von der Regeleinheit vorgegebenen Ultrafiltrationsrate UFR(t) mit einem unteren Grenzwert für die Ultrafiltrationsrate aufweist, wobei die Überwachungseinheit derart ausgebildet ist, daß die Ultrafiltrationsrate UFR(t) nur dann auf den oberen Grenzwert $UFR_{lim}$ begrenzt wird, wenn die Ultrafiltrationsrate UFR größer als der untere Grenzwert ist.

**Claims**

1. Safety device for an extracorporeal blood treatment device, which exhibits

   a blood supply line (7), which leads from the patient to the inlet of a first chamber (3) of an exchange unit (1), divided by a semi-permeable membrane (2) into the first and a second chamber (3, 4), and a blood discharge line (8) which leads from an outlet of the first chamber to the patient,

   an ultrafiltration device (18, 19, 20) for extracting a predetermined total ultrafiltrate volume ($UFV_{tot}$ from the second chamber of the exchange unit, at an ultrafiltration rate UFR(t) during a predetermined treatment time UFT,

   a measuring unit (21) for determining the blood volume BV or a measured value of the patient correlating to the blood volume, and

   a control unit (23) for predetermining an ultrafiltration rate UFR(t) as a function of the blood volume BV, the safety device (28) exhibiting:

   a unit (29) for predetermining an upper limit value for the ultrafiltration rate UFR(t) and a monitoring unit (30) for limiting the ultrafiltration rate UFR(t) predetermined by the control unit (23) to the upper limit value $UFR_{lim}$ when the ultrafiltration rate UFR(t) predetermined by the control unit reaches the upper limit value $UFR_{lim}$, **characterised in that** the unit for predetermining an upper limit value for the ultrafiltration rate UFR(t) is a calculating unit which is designed so that the upper limit value can be determined from the predetermined total ultrafiltrate volume $UFV_{tot}$ and the predetermined treatment time UFT.

2. Safety device according to claim 1, **characterised in that** the calculating unit (29) is designed so that the mean ultrafiltration rate UFRM required to extract the predetermined total ultrafiltrate volume $UFV_{tot}$ within the treatment time UFT can be determined by forming the quotient of the predetermined total ultrafiltrate volume $UFV_{tot}$ and the predetermined treatment time UFT, and **in that** the upper limit value $UFR_{lim}$ can be determined by multiplying the mean ultrafiltration rate UFRM by a factor $\alpha$.

3. Safety device according to claim 1, **characterised in that** the calculating unit (29) is designed so that an upper limit value, which decreases as the treatment time increases, can be determined for the ultrafiltration rate $UFR_{lim}(t)$.

4. Safety device according to claim 1, **characterised in that** the calculating unit (29) is designed so that an upper limit value, which decreases as the ultrafiltrate volume UFV increases, can be determined for the ultrafiltration rate $UFR_{lim}(t)$.

5. Safety device according to claim 3 or 4, **characterised in that** the calculating unit (29) is designed so that the mean ultrafiltration rate UFRM required to extract the predetermined total ultrafiltrate volume $UFV_{tot}$ within the treatment time UFT can be determined by forming the quotient of the predetermined total ultrafiltrate volume $UFV_{tot}$ and the predetermined treatment time UFT, and **in that** the upper limit value $UFR_{lim}(t, \alpha)$ at a certain time t can be determined according to the following equation

$$UFR_{lim}(t,\alpha)=[\alpha \cdot UFRM-(\alpha-1) \cdot UFRM \cdot \frac{t}{UFT}] \cdot \Phi(1-\frac{t}{UFT})+UFRM \cdot \Phi(\frac{t}{UFT}-1)$$

   where $\Phi(t) = 0$ for $t < 0$ and $\Phi(t) = 1$ for $t \geq 0$.

6. Safety device according to claim 1, **characterised in that** the calculating unit (29) is designed so that the residual ultrafiltration rate UFRR(t) at which the ultrafiltrate volume UFV can be extracted at a certain time in the remaining period of the treatment time UFT can be determined from the predetermined total ultrafiltrate volume $UFV_{tot}$ and the predetermined treatment time UFT, according to the following equation

$$UFRR(t) = (UFV_{tot} - UFV(t)) / UFT\text{-}t,$$

   and **in that** the upper limit value $UFT_{lim}(t, \alpha)$ at this time t can be determined by multiplying the residual ultrafiltration rate UFRR(t) by a factor $\alpha$.

7. Safety device according to claim 6, **characterised in that** a warning device (34) is provided which exhibits a comparison device for comparing the residual ultrafiltration rate UFRR(t) with the mean ultrafiltration rate UFRM, the warning device being designed so that a warning signal is emitted after the mean ultrafiltration rate UFRM is exceeded.

8. Safety device according to claim 6, **characterised in that** a warning device (34) is provided with exhibits a comparison device for comparing the residual ultrafiltration rate UFRR(t) with the upper limit value $UFR_{lim}$ for the ultrafiltration rate, the warning device being designed so that a warning signal is emitted after the upper limit value $UFR_{lim}$ is exceeded.

9. Safety device according to one of claims 1 to 8, **characterised in that** the monitoring unit (30) exhibits a comparison device for comparing the ultrafiltration rate UFR(t) predetermined by the control unit with a lower limit value for the ultrafiltration rate, the monitoring unit being designed so that the ultrafiltration rate UFR(t) is limited to the upper limit value $UFR_{lim}$ only when the ultrafiltration rate UFR is greater than the lower limit.

**Revendications**

1. Dispositif de sécurité pour un appareil extracorporel de traitement du sang, qui présente

   une conduite d'arrivée du sang (7), qui conduit à partir du patient à l'entrée d'une première chambre (3) d'une unité d'échange (1) séparée par une membrane semi-perméable (2) en une première et une deuxième chambre (3, 4), et à une conduite de sortie du sang (8), qui conduit au patient depuis la sortie de la première chambre, un mécanisme d'ultrafiltration (18, 19, 20) pour le prélèvement d'un volume total donné du produit d'ultrafiltration total $VUF_{tot}$ en dehors de la deuxième chambre de l'unité d'échange avec un taux d'ultrafiltration TUF(t) pendant un temps donné de traitement TUF,
   une unité de mesure (21) pour le référencement du volume sanguin VS ou d'une autre valeur en corrélation avec le volume sanguin du patient et
   une unité de calcul (23) pour l'établissement du taux d'ultrafiltration TUF(t) en relation avec le volume sanguin VS, dans laquelle le dispositif de sécurité (28) présente :

   une unité (29) pour le référencement d'une valeur seuil maximale pour le taux d'ultrafiltration TUF(t) et une unité de surveillance (30) pour limiter le taux d'ultrafiltration TUF(t) indiqué préalablement par l'unité de calcul (23) à la valeur seuil maximale de $TUF_{lim}$ lorsque le taux d'ultrafiltration TUF(t) indiqué préalablement par l'unité de calcul atteint la valeur seuil maximale $TUF_{lim}$, **caractérisé en ce que** l'unité pour le référencement d'une valeur seuil maximale pour le taux d'ultrafiltration TUF(t) est une unité informatisée, formée de telle façon que la valeur seuil maximale est définissable à partir du volume total du produit d'ultrafiltration donné $VUF_{tot}$ et du temps de traitement TUF donné.

2. Dispositif de sécurité selon la revendication 1, **caractérisé en ce que** l'unité de calcul (29) est formée de telle façon que, par la détermination du quotient du volume total du produit d'ultrafiltration $VUF_{tot}$ et du temps de traitement TUF donné, le taux d'ultrafiltration moyen TUFM, qui est nécessaire pour le prélèvement du volume total du produit d'ultrafiltration $VUF_{tot}$ pendant le temps de traitement TUF, peut être déterminé, de même que la valeur seuil maximale $VUF_{lim}$, par la multiplication du taux d'ultrafiltration moyen TUFM par un facteur $\alpha$.

3. Dispositif de sécurité selon la revendication 1, **caractérisé en ce que** l'unité de calcul (29) est formée de telle façon qu'une valeur seuil maximale pour le taux d'ultrafiltration $TUF_{lim}(t)$ qui décroît avec un temps de traitement croissant est définissable.

4. Dispositif de sécurité selon la revendication 1, **caractérisé en ce que** l'unité de calcul (29) est formée de telle façon qu'une valeur seuil maximale pour le taux d'ultrafiltration $TUF_{lim}(t)$ qui décroît avec un volume d'ultrafiltration VUF croissant est définissable.

5. Dispositif de sécurité selon la revendication 3 ou 4, **caractérisé en ce que** l'unité de calcul (29) est formée de telle façon que, par la détermination du quotient du volume total du produit d'ultrafiltration $VUF_{tot}$ et du temps de traitement TUF donné, le taux d'ultrafiltration moyen TUFM, qui est nécessaire pour le prélèvement du volume

**EP 0 960 625 B1**

total du produit d'ultrafiltration $VUF_{tot}$ pendant le temps de traitement TUF, peut être déterminé à un moment t donné d'après l'équation suivante de la valeur seuil maximale $VUF_{lim}(t, \alpha)$,

$$VUF_{lim}(t,\alpha)=[\alpha.TUFM-(\alpha-1)-TUFM.t/TUF].\phi(1-t)+TUFM.\phi(t/TUF-1)$$

où $\phi(t)=0$ pour $t<0$ et $\phi(t)=1$ pour $t\geq0$.

6. Dispositif de sécurité selon la revendication 1, **caractérisé en ce que** l'unité de calcul (29) est formée de façon à ce que le taux d'ultrafiltration restant TUFR soit définissable à partir du volume total du produit d'ultrafiltration $FUV_{tot}$ donné et du temps de traitement TUF donné d'après l'équation suivante

$$TUFR(t)=(FUV_{tot}-FUV(t))/(TUF-t)$$

grâce à laquelle le volume d'ultrafiltration VUE peut être prélevé à un moment déterminé dans la période restante du temps de traitement TUF, et qui est définissable par la multiplication du taux d'ultrafiltration restant TUFR(t) par un facteur $\alpha$ de la valeur seuil maximale VUFlim(t,$\alpha$) à ce moment t.

7. Dispositif de sécurité selon la revendication 6, **caractérisé en ce qu'**un dispositif d'alerte (34) est prévu, qui présente un dispositif de comparaison pour la comparaison du taux d'ultrafiltration restant TUFR(t) et du taux d'ultrafiltration moyen TUFM, dans lequel le dispositif d'alerte est formé de façon à ce qu'un signal soit émis en cas de dépassement du taux d'ultrafiltration moyen TUFM.

8. Dispositif de sécurité selon la revendication 6, **caractérisé en ce qu'**un dispositif d'alerte (34) est prévu, qui présente un dispositif de comparaison pour la comparaison du taux d'ultrafiltration restant TUFR(t) et de la valeur seuil maximale d'ultrafiltration $VUF_{lim}$, dans lequel le dispositif d'alerte est formé de façon à ce qu'un signal soit émis en cas de dépassement du taux d'ultrafiltration maximal $VUF_{lim}$.

9. Dispositif de sécurité selon l'une quelconque des revendications 1 à 8, **caractérisé en ce que** l'unité de surveillance (30) présente un dispositif de comparaison pour la comparaison du taux d'ultrafiltration TUF(t) donné préalablement par l'unité de calcul et une valeur seuil minimale pour le taux d'ultrafiltration, dans lequel l'unité de surveillance est formée de telle façon que le taux d'ultrafiltration TUF(t) n'est limité à la valeur seuil maximale $VUF_{lim}$ que quand le taux d'ultrafiltration TUF est plus élevé que la valeur seuil minimale.

Fig.1

**Fig. 2**

Eingabe:

$UFV_{ges}$
$UFT$
$UFR_{min}$

$UFR(t) \geq UFR_{min}$?

nein

ja

Limitierung aktiviert?

nein

ja

$UFR(t) \geq UFR_{lim}$?

nein

ja

Limitierung von
$URF(t)$ auf $UFR_{lim}$

UFR(t,1.7)
UFR(t,2.0)
UFR(t,2.3)

t

Fig.3

UFV(t,1.7)(II)
UFV(t,2.0)(III)
UFV(t,2.3)(IV)
UVO(t)(I)

t

Fig.4

$\underline{UFV}(t,1.7)\,(I)$
$\underline{UFV}(t,2.0)\,(II)$
$\underline{UFV}(t,2.3)\,(III)$ 0.5
$\underline{UVO}(t)\,(IV)$

Fig. 5

Fig. 6